# EUROPÄISCHE PATENTSCHRIFT

(11) **EP 0 725 133 B1**
(45) Veröffentlichungstag und Bekanntmachung des Hinweises auf die Patenterteilung: **09.12.1998**
(21) Anmeldenummer: 95113833.8
(22) Anmeldetag: 04.09.1995
(51) Int. Cl.: C12M 1/00

(54) **Klimaschrank, Karussell und Verwendung des Karussells in einem Klimaschrank**
Incubator, carrousel and use of the carrousel in an incubator
Incubateur, carrousel et utilisation du carrousel dans un incubateur

(30) Priorität: 06.02.1995 CH 328/95
(43) Veröffentlichungstag der Anmeldung: 07.08.1996
(73) Patentinhaber: LICONIC AG, FL-9493 Mauren (LI); Heraeus Instruments GmbH & Co. KG, 63450 Hanau (DE)
(72) Erfinder: Malin, Cosmas, FL-9493 Mauren (LI); Sawatzki, Harry, FL-9440 Schaan (LI); Gentsch, Jürg, CH-4104 Oberwil (CH); Helwig, Günther, D-63457 Hanau (DE)
(74) Vertreter: Hotz, Klaus, Dipl.-El.-Ing./ETH Patentanwalt

(56) Entgegenhaltungen:
- EP-A- 0 293 782
- GB-A- 501 110
- GB-A- 926 541
- GB-A- 1 112 919

## Beschreibung

Die Erfindung bezieht sich auf einen Klimaschrank nach dem Oberbegriff des Patentanspruchs **1**, auf ein Karussell nach dem Oberbegriff des Patentanspruchs **13** und auf eine Verwendung des Karussells nach dem Oberbegriff des Patentanspruchs **19**.

Klimaschränke dienen zum definierten Erzeugen von Klimaverhältnissen. Insbesondere in Anwendungen, in denen extreme Luftfeuchtigkeiten garantiert sein müssen, werden sehr hohe Anforderungen an die Homogenität der Temperaturverteilung im Klimaschrankinneren sowie an die thermische Isolation des Klimaschranks gestellt. Häufig weisen solche Klimaschränke zusätzlich doppelte Wandungen mit einem Zwischenraum auf, in dem sich ein temperiertes Medium (Wasser, Luft) befindet. Dies bedingt grosse Wandstärken von mehreren Zentimetern. In Anwendungen mit automatischer Beschickung geschieht der Zugriff zum Klimaschrank nicht nur automatisch, sondern häufig auch manuell durch den Benutzer. Besonderes Augenmerk muss auf die beengten Platzverhältnisse bedingt durch die einerseits vorgesehene automatische Beladung, meistens durch Roboter und andererseits durch den Wunsch nach optimaler Platznutzung gerichtet werden. Von grosser Bedeutung ist ferner die Sauberkeit im Klimaschrankinneren sowie die flexible Raumaufteilung desselben.

Beispielsweise ist in der **EP 0 293 782 B1** ein Inkubator mit einem drehbaren Schalenlager und einer Hilfstüre beschrieben. Diese Anordnung ist für Anwendungen mit erhöhten Klimaanforderungen nicht geeignet da die vorgehene Hilfstüre ein störungsarmes Überbrücken grosser Wandstärken nicht zulässt. Ferner ist die Hilfstüre in die Servicetüre integriert, was den simultanen Roboter- und Benutzerzugriff verunmöglicht. Die Entnahme des Schalenlagers ist schwierig, da die Drehachse des Schalenlagers über die obere Wandung des Inkubators hinausragt. Die Anordnung des Schalenlagers und des Antreibsmechanismus erfordern im weiteren die Verlagerung des Antriebsmechanismus aus dem Inneren des Klimaschrankes, was den Einsatz in preiskritischen Anwendungen erschwert. Der Aufbau des Schalenlagers ist zudem wenig stabil und erlaubt keine hohe Präzision, keine exakte Positionierung der Schalen und nur niedrige Beschleunigungen.

Aus der GB-926 541 ist sodann ein Klimaschrank mit einem Karussell bekannt, das in den Klimaschrank eingesetzt und wieder herausgenommen werden kann. Das Karussell kann in mehreren Etagen Lagergutstücke aufnehmen, und zwar auf ringförmigen Böden bzw. Scheiben. Diese sind durch sie distanzierende Teile voneinander beabstandet. Die distanzierenden Teile sind vertikale Wände, die fest mit den ringförmigen Scheiben verbunden sind und deren radiale Abmessungen gleich den radialen Abmessungen der ringförmigen Scheiben sind. Der Nachteil dieses Karussells ist darin zu sehen, dass die Böden und Wände, die aus Platzgründen und wegen der Bewegung des Karussells leicht ausgebildet sein sollten, ein nicht sehr stabiles Gestell bilden. Da die Wände und die Böden fest verbunden sind, ist dieses Gestell auch nicht an verschiedenen Grössen von Lagergutstükken anpassbar.

Die Aufgabe der Erfindung besteht ferner darin, ein Karussell der eingangs genannten Art vorzuschlagen, das im zusammengebauten Zustand starr ist, sich jedoch in einfacher Weise an verschieden hohe Lagergutstücke anpassen lässt.

Im weiteren besteht die Aufgabe der Erfindung darin, die Verwendung eines solchen Karussells aufzuzeigen.

Diese Aufgabe wird gelöst
- an einem Klimaschrank der eingangs genannten Art durch die Merkmale des kennzeichnenden Teils des Patentanspruchs **1**;
- an einem Karussell der eingangs genannten Art durch die Merkmale des kennzeichnenden Teils des Patentanspruchs **13**; und
- für die Verwendung der eingangs genannten Art durch die Merkmale des kennzeichnenden Teils des Patentanspruchs **19**.

Bevorzugte Weiterbildungen und vorteilhafte Ausführungsbeispiele des erfindungsgemässen Klimaschrankes und des erfindungsgemässen Karussells werden durch die abhängigen Patentansprüche **2** bis **12** und **14** bis **18** beschrieben.

Der neue Klimaschrank weist somit die folgenden Merkmale auf:
- er umfasst eine konventionelle Türe auf der Benutzerseite und eine steuerbare Türe auf der Beschickungsseite;
- die steuerbare Türe ist einerseits auf einem ersten Führungsystem senkrecht zur Beschickungsseite bewegbar gelagert und andererseits auf einem zweiten Führungssystem parallel zur Beschickungsseite bewegbar gelagert;
- das Karussell ist an seiner Unterseite mittels eines Lagers abgestützt;
- das Karussell ist aus Tellerscheiben und Distanzhülsen aufgebaut;
- auf den Tellerscheiben sind auf der Telleroberfläche aufbauende Positionierelemente vorgesehen;
- die Kupplung zwischen Karussell und Positionierantrieb ist im Klimaschrankinneren angeordnet; und
- der Positionierantrieb ist sowohl von Benutzer als auch von übergeordneten System steuerbar.

Die Vorteile der Erfindung sind darin zu sehen, dass der neue Klimaschrank
- für Roboteranwendungen eine optimale Anordnung von Benutzer- und Beschickungstüre aufweist;
- eine Überbrückung grosser Wandstärken bei minimaler Störung der Isolation bzw. des Temperiermediums erlaubt;
- präzise Positionierung durch hohe Stabilität des Karussells möglich ist;
- eine definierte Lage von Objekten durch vorteilhafte Ausgestaltung der Tellerscheiben und Positionierelemente erlaubt;
- maximale Platznutzung auf der Beschickungsseite gewährleistet;
- infolge minimalen Platzbedarfs bei Türöffnung ungehinderte Bewegung des Beschickungsroboter unabhängig von der Türstellung ermöglicht;
- leichtes Entfernen des Karussells bei Wartungsarbeiten gestattet;
- komfortablen und sicheren Zugriff für den Benutzer gewährleistet;
- optimale Raumnutzung des Klimaschrankinneren garantiert;
- so konstruiert ist, dass der Karussellaufbau jederzeit neuen Bedürfnissen angepasst werden kann; und
- dank des stabilen Aufbaus des Karussells hohe Beschleunigunen, wie sie beispielsweise bei Schüttelbewegungen notwendig sind, ermöglicht.

Die Vorteile des neuen Karussells ergeben sich aus den diesbezüglichen vorteilhaften Eigenschaften des Klimaschrankes. Besonders vorteilhaft ist es, dass sich das Karussell sowohl als Lagervorrichtung sowie zur Verwendung in einer Einrichtung wie beispielsweise in einem Klimaschrank eignet.

Im folgenden wird die Erfindung anhand mehrerer in den Figuren der Zeichnung dargestellter Ausführungsbeispiele beschrieben. Es zeigt:
- **Fig. 1**: einen erfindungsgemässen Klimaschrank mit einem ersten Ausführungsbeispiel der steuerbaren Türe, wobei sich der Positionierantrieb im Nutzraum befindet;
- **Fig. 2**: einen weiteren Klimaschrank mit einem zweiten Ausführungsbeispiel der steuerbaren Tür, von oben;
- **Fig. 3a**: den in Fig. 1 dargestellten Klimaschrank mit dem sich im Nutzraum befindlichen Positionierantrieb, von der Seite;
- **Fig. 3b**: einen erfindungsgemässen Klimaschrank mit einem sich ausserhalb des Nutzraums befindlichen Positionierantrieb, von der Seite;
- **Fig. 4**: ein Ausführungsbeispiel einer Tellerscheibe, von oben;
- **Fig. 5**: ein Ausführungsbeispiel des Karussells mit Distanzhülsen, teilweise geschnitten, von der Seite;
- **Fig. 6a**: ein erstens Ausführungsbeispiel eines Details der Tellerscheibe, von oben;
- **Fig. 6b**: das in Fig. 6a dargestellte Detail der Tellerscheibe, ausschnittweise, in einem vertikalen Teilschnitt;
- **Fig. 7a**: ein zweites Ausführungsbeispiels des Details der Tellerscheibe, von oben; und
- **Fig. 7b**: das in Fig. 7a dargestellte Detail der Tellerscheibe, ausschnittweise, in einem vertikalen Teilschnitt.

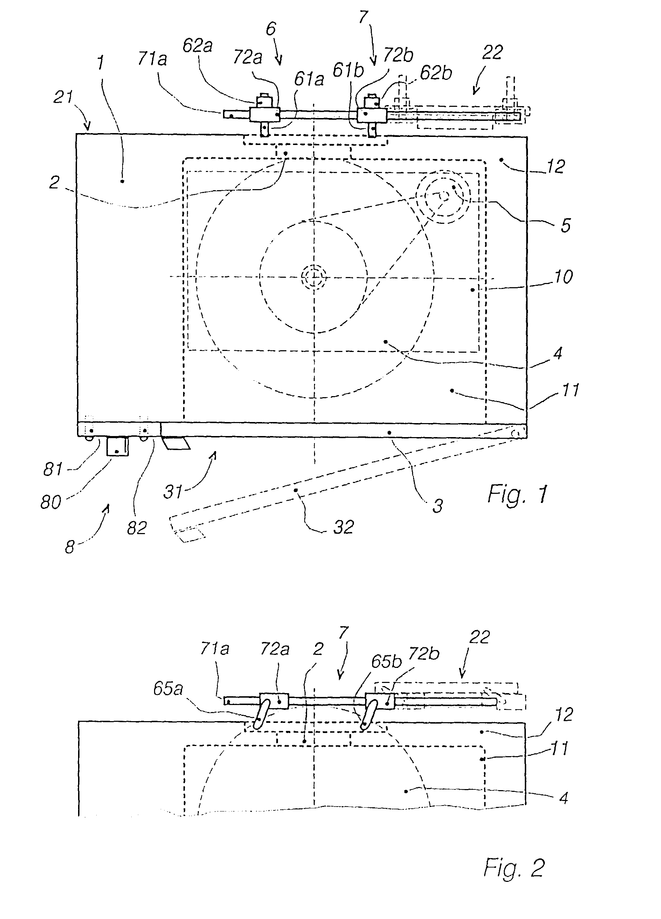

In der Fig. **1** ist die Aufsicht eines Klimaschranks **1** mit einer steuerbaren Türe **2** auf einer Beschickungsseite **21**, einer konventionellen Türe **3** auf einer Benutzerseite **31** und einem Nutzraum **11** dargestellt. Im Nutzraum **11** ist ein im weiteren als Karussellaufbau **4** bezeichnetes Karussell vorgesehen, der über einen Positionierantrieb **5** rotiert bzw. positioniert wird. Der Karussellaufbau **4** und der Positionierantrieb **5** sind auf einer Platte **10** angeordnet. Eine Wandung **12** trennt den Nutzraum **11** von der Aussenwand des Klimaschranks **1**. Auf der Benutzerseite **31** ist ferner ein Positionswähler **80** für die halbautomatische Positionierung des Karussellaufbaus **4** angebracht. Das Bezugszeichen **32** bezeichnet die geöffnete konventionelle Türe **3**, das Bezugszeichen **22** die steuerbare Türe **2** in vollständig geöffnetem Zustand.

Die steuerbare Türe **2** ist auf einem ersten linearen Führungssytem **6**, das die steuerbare Türe **2** senkrecht zur Beschickungsseite **21** bewegt, befestigt. Das erste lineare Führungssystem **6** besteht dabei aus Hubschienen **61a, 61b** und aus Hubbüchsen **62a, 62b**. Mittels des ersten linearen Führungssystems **6** kann die steuerbare Türe **2** eine Hubbewegung senkrecht zur Beschickungsseite **21** vollziehen. Das erste lineare Führungssystem **6** ist seinerseits auf einem zweiten linearen Führungssystem **7** befestigt, das parallel zur Beschickungsseite **21** verläuft. Auf dem zweiten linearen Führungssystem **7** kann die steuerbare Türe **2** parallel zur Beschikkungsseite **21** bewegt werden, indem Schiebebüchsen **72** auf mindestens einer Schiebeschiene **71** verfahren. Das zweite lineare Führungssystem **7** ist an der Beschickungsseite **21** des Klimaschranks **1** befestigt. Durch die obige Anordnung kann man grosse Wandungen **12** überbrücken bzw. grosse Wandstärken in der steuerbaren Türe **2** realisieren, ohne eine grosse Auslagerung der steuerbaren Türe **2** oder grosse Türspalten zwischen der steuerbaren Türe **2** und der Wandung **12** in Kauf nehmen zu müssen.

Mit **8** sind Positionswählelemente bezeichnet, die es dem Benutzer erlauben, manuellen Zugriff auf den Karussellaufbau **4** zu nehmen. Der Positionswähler **80** ist so gestaltet, dass er eine ortsgerechte Positionierung des Karussellaufbaues **4** ermöglicht. Dabei erkennt dann der Benutzer an einer ersten Signallampe **81** bzw. einer zweiten Signallampe **82**, wenn der Karussellaufbau **4** positioniert und damit ein manueller Zugriff möglich ist.

In **Fig. 2** ist ein zweites Ausführungsbeispiel zur steuerbaren Türe **2** dargestellt Bei diesem Ausführungsbeispiel bilden Kipphebel **65a** und **65b** das erste Führungssystem, mit dem die steuerbare Türe **2** senkrecht zur Beschickungsseite bewegbar ist. Die Bewegung durch die Kipphebel **65a** und **65b** setzt sich aus zwei Komponenten zusammen, wobei eine dieser Komponenten in senkrechter Richtung zur Beschickungsseite verläuft Dieses erste Führungssystem ist beispielsweise mittels nicht dargestellten Kurvenscheiben steuerbar. Das zweite Führungssystem besteht aus den Schiebebüchsen **72a, 72b** und der Schiebeschiene **71a** und ermöglicht die Bewegung der steuerbaren Türe **2** parallel zur Beschickungsseite.

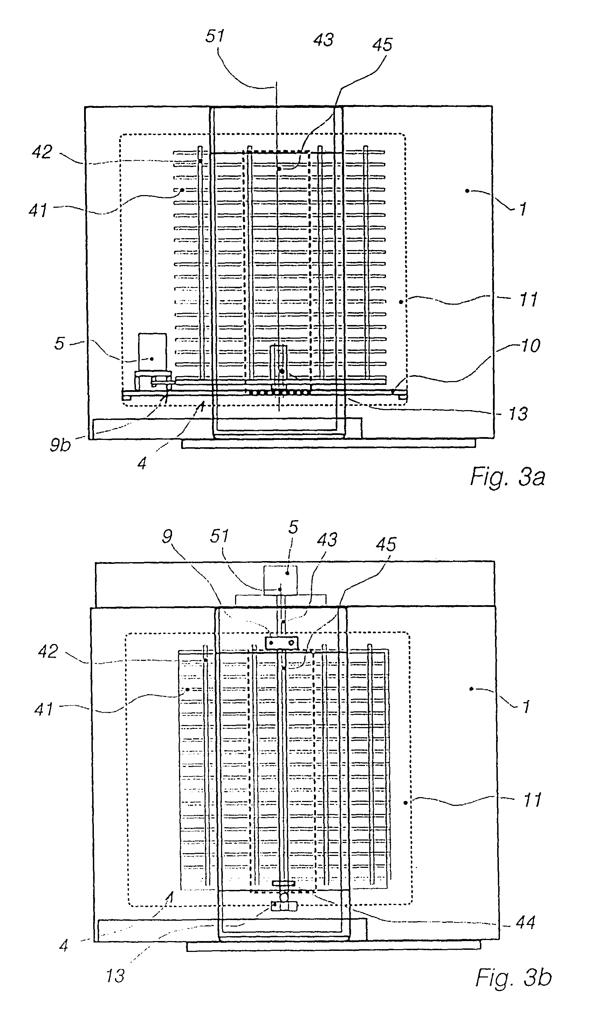

In **Fig. 3a** ist eine Ansicht der Beschickungsseite **21** des Klimaschrankes **1** dargestellt. Der Karussellaufbau **4** und der Positionierantrieb **5** sind auf einer Platte **10** angeordnet, wobei die Lagervorrichtung für den Karussellaufbau **41** mit **13** bezeichnet ist. Eine Verbindungsstelle **9b** zwischen dem Karussellaufbau **4** und dem Positionierantrieb **5** befindet sich innerhalb des Nutzraums **11** und - im Grundriss gesehen - ausserhalb des Bereichs des Karussellaufbaus **4**, Dadurch, dass der Positionierantrieb **5** gemeinsam mit dem Karussellaufbau **4** auf der Platte **10** angeordnet ist, kann der gesamte Karussellaufbau **4** in einfacher Weise mit dem Platte **10** dem Nutzraum **11** entnommen werden. Der Karassellaufbau ist im weiteren so gestaltet, dass er sich auch zur Verwendung ausserhalb des Klimaschrankes **1** eignet.

In **Fig. 3b** ist ebenfalls eine Ansicht, von der Beschickungsseite **21** her, des Klimaschranks **1** dargestellt. Der Karussellaufbau **4** liegt am Boden des Nutzraums **11** auf, wobei die Lagerung wiederum mit **13** bezeichnet ist. Durch diese Anordnung ist eine optimale Platznutzung des Nutzraums **11** möglich. Die Verbindung zwischen dem Karussellaufbau **4** und dem Positionierantrieb **5** wird durch eine Kupplung **9** gebildet. Der Positionierantrieb **5** weist bei diesem Ausführungsbeispiel dieselbe Rotationsachse auf wie der Karussellaufbau **4**. Eine Antriebswelle **43** des Positionierantriebs **5**, über den auch die Richtungsänderung realisiert wird, ist über die Kupplung **9** mit einer Trägerwelle **45** des Karussellaufbaus **4** verbunden. Die Kupplung **9** ist innerhalb des Nutzraums **11** angeordnet. Dies erlaubt einen einfachen Ein- bzw. Ausbau des Karussellaufbaus **4**.

Der Karussellaufbau **4** ist aus übereinanderliegenden Tellerscheiben **41** aufgebaut.

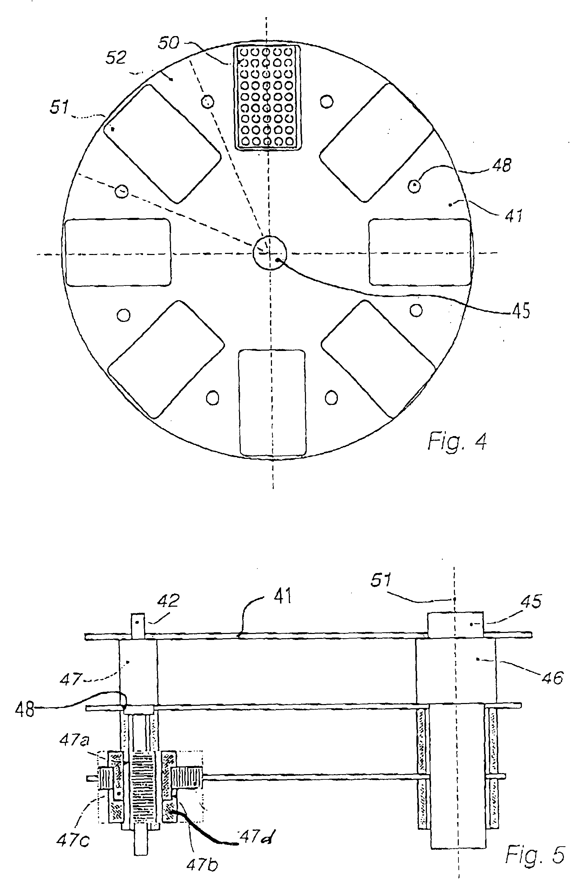

**Fig. 4** zeigt in Aufsicht ein Ausführungsbeispiel einer Tellerscheibe **41**. Die Tellerscheibe **41** weist neben einer zur Trägerwelle **45** konzentrischen Zentrumsbohrung wenigstens drei Stützbohrungen **48** in der Randzone der Tellerscheibe **41** auf. Die Tellerscheibe **41** ist sternförmig in Segmente **52** aufgeteilt. Die Segmente **52** dienen der Aufnahme jeweils eines rechteckigen Lagergutstücks **50**. Das Lagergutstück **50** wird auf der Tellerscheibe **41** durch Positionierelemente **51** gehalten. In dem Ausführungsbeispiel der **Fig. 4** sind die Positionierelemente **51** als in die Tellerscheibe **41** eingelassene Vertiefungen mit der Form des Umrisses der Aufsicht des Lagergutstücks **50** vorgesehen.

**Fig. 5** stellt eine teilweise Schnittdarstellung des Karussellaufbaus **4** dar. Der Karussellaufbau **4** weist neben der Trägerwelle **45**, welche die Rotationsachse bildet, wenigstens drei Karussellstützen **42** auf, die parallel zur Trägerwelle **45** und senkrecht zu den Tellerscheiben **41** verlaufen. Die Trägerwelle **45** ist im Rotationszentrum des Karussellaufbaus **4** angeordnet. Die Tellerscheiben **41** sind konzentrisch zur Trägerwelle **45** in definierten Abständen übereinander als Etagen angeordnet. Die Abstände zwischen den Tellerscheiben **41** werden durch Distanzhülsen **47** gebildet. Die Distanzhülsen **47** weisen eine Längsbohrung **47a**, eine Zentrierbohrung **47b** sowie einen Zentrierkragen **47c** auf. Der Innendurchmesser der Zentrierbohrung **47b** ist gleich dem Aussendurchmesser des Zentrierkragens **47c**. Der Durchmesser der Stützbohrungen **48** in der Tellerscheibe **41** ist gleich dem Durchmesser der Zentrierbohrung **47b**. Der Zentierkragen **47c** jeder Distanzhülse **47** ist durch die Stützbohrung **48** einer Tellerscheibe **41** und den Zentrierkragen **47c** einer weiteren Distanzhülse **47** geführt. So wird eine Kette aus Tellerscheiben **41** und Distanzhülsen **47** gebildet, in der jeweils eine Tellerscheibe **41**, geführt durch den Zentrierkragen **47c**, zwischen zwei Distanzhülsen **47** liegt. Im Innern der Kette verläuft entlang der Längsbohrungen **47a** eine Spannstange **42**, die die Distanzhülsen **47** bzw. Tellerscheiben **41** an den beiden Enden der Kette längsseitig auf nicht dargestellte Weise verspannt. Die Zentrierbohrung kann auch an einer das eine Ende der Distanzhülse bildenden Zentrierhülse **47d** angeordnet sein.

Um optimale Stabilität des Karussellaufaus **4** und Planität der Tellerscheiben **41** zu erreichen ist es vorteilhaft, den Karussellaufbau **4** zusätzlich im Zentrum zu verspannen. In dem vorliegenden Ausführungsbeispiel sind zwischen den Tellerscheiben **41** Distanzröhrchen **46** angeordnet. Die Distanzröhrchen **46** weisen die gleiche Länge wie die Distanzhülsen **47** einer jeweiligen Etage auf und umschlingen die Trägerwelle **45**. Die Distanzröhrchen **46** bzw. Tellerscheiben **41** liegen an der Unterseite des Karussellaufbaus **4** auf einem in **Fig. 3** dargestellten Flansch **44** auf, der mit der Trägerwelle **45** verbunden ist. Die Distanzröhrchen **46** sind auf der Oberseite des Karussellaufbaus **4** mittels der Kupplung **9** gegen den Flansch **44** verspannt.

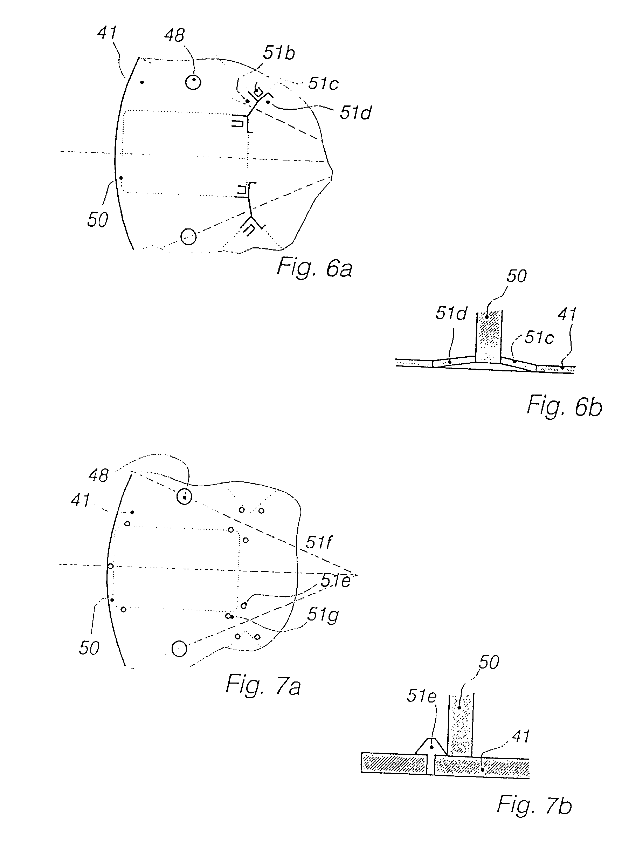

**Fig. 6a** zeigt ausschnittweise eine Detaildarstellung eines Ausführungsbeispiels der Tellerscheibe **41** aus **Fig. 4**, mit speziell ausgeführten Positionierelementen **51**. Diese sind dabei als über die Oberfläche der Tellerscheibe **41** hinausragende Laschen **51b - 51d** ausgebildet sind. Das Lagergutstück **50** wird in diesem Ausführungsbeispiel an der Innen- bzw. Aussenseite seiner Seitenwände durch die Positionierelemente **51c** und **51d** radial, durch die Positionierelemente **51b** und **51c** tangential gehalten.

**Fig. 6b** verdeutlicht in einer ausschnittsweisen Schnittdarstellung die Konstruktion aus **Fig. 5a**.

**Fig. 7a** stellt eine Variante der Ausbildung für die Halterung der Lagerstücke **50** mit Positionierelememten **51** dar. Die Positionierelemente **51** sind in diesem Ausführungsbeispiel in die Tellerscheibe **41** so eingepresste Stifte **51e** - **51f**, dass ein Teil der eingepressten Stifte **51e** - **51f** über die Oberfläche der Tellerscheibe **41** hinausragt. Die Stifte **51e** und **51f** sind dabei so angeordnet, dass sie das Lagergutstück radial, die Stifte **51g** tangential an den Seitenwänden des Lagergutstücks **50** halten.

**Fig. 7b** verdeutlicht in einer ausschnittsweisen Schnittdarstellung die Konstruktion aus **Fig. 6a**.

Um gleiche Klimaverhältnisse für sämtliche Lagergutstücke **50** unabhängig von Ihrem Ort innerhalb des Nutzraums zu gewährleisten, führt der Karussellaufbau **4** eine dauernde Rotationsbewegung aus. In Fällen wo eine Durchmischung einer Flüssigkeit in den Lagergutstücken erforderlich ist, wird ein Schütteln durch intermittierende Richtungsänderung der Rotationsbewegung des Karussellaufbaus **4** erzeugt. Durch eine kleine Abweichung der beiden Zeitdauern für die Vor- bzw. Rückbewegung ist die Schüttelbewegung durch eine Rotationsbewegung in diesem Falle überlagert. Die Bedienelemente, d.h. die Positionswählelemente **8** können auch ein übergeordnetes System beinhalten, dass dem Benützer erlaubt, Terminkalender und Inhalt der Lagergutstücke **50** so zu verknüpfen, dass der Bediener beispielsweise festlegen kann, wann und wie lange die zu behandelnden Proben im Klimaschrank verbleiben sollen.

## Patentansprüche

1. Klimaschrank, welcher automatisch von einem übergeordneten System beschickbar ist und eine steuerbare und eine konventionelle Türe sowie ein sich im Klimaschrankinneren befindliches Karussell aufweist, das über eine lösbare Kupplung mit einem Positionierantrieb verbunden ist,
**dadurch gekennzeichnet,**
dass
- die konventionelle Türe **(3)** auf einer Benutzerseite **(31)** und die steuerbare Türe **(2)** auf einer Beschickungsseite **(21)** angeordnet ist,
- die steuerbare Türe **(2)** einerseits auf einem ersten Führungssystem **(6)** senkrecht zur Beschickungsseite **(21)** bewegbar gelagert ist und andererseits auf einem zweiten Führungssystem **(7)** parallel zur Beschickungsseite **(21)** bewegbar gelagert ist,
- das Karussell **(4)** auf einem im Boden des Klimaschrankinneren angeordneten Lager **(13)** abgestützt ist,
- das Karussell **(4)** aus einer Trägerwelle **(45)**, wenigstens zwei Tellerscheiben **(41)** und wenigstens drei Distanzhülsen **(47)** aufgebaut ist und die Trägerwelle **(45)** und die Distanzhülsen **(47)** senkrecht auf den Tellerscheiben **(41)** angeordnet sind,
- auf den Tellerscheiben **(41)** aus der Telleroberfläche aufbauende Positionierelemente **(51)** vorgesehen sind,
- die Kupplung **(9)** zwischen dem Karussell **(4)** und dem Positionierantrieb **(5)** im Klimaschrankinneren angeordnet ist,
- der Positionierantrieb **(5)** sowohl von Benutzer über Bedienelemente als auch vom übergeordneten System steuerbar ist.

2. Klimaschrank nach Patentanspruch **1**,
**dadurch gekennzeichnet**,
dass die Führungssysteme **(6, 7)** Linearführungssysteme sind, wobei das erste Linearführungssystem **(6)** senkrecht zu der Beschickungsseite **(21)** bewegbar auf dem zweiten, parallel zu der Beschikkungsseite **(21)** angeordneten Linearführungssystem **(7)** angeordnet ist.

3. Klimaschrank nach Patentanspruch **1**,
**dadurch gekennzeichnet,**
dass das erste Führungssystem **(6)** ein Rotationsführungssystem und das zweite Führungssystem **(7)** ein Linearführungssystem ist, wobei das Rotationsführungssystem die steuerbare Türe **(2)** senkrecht zu der Beschickungsseite **(21)** bewegt und auf dem zweiten, parallel zu der Beschickungsseite **(21)** angeordneten Linearführungssystem **(7)** angeordnet ist.

4. Klimaschrank nach Patentnspruch **1**,
**dadurch gekennzeichnet,**
dass die Distanzhülsen mindestens drei äussere Distanzhülsen **(47)** umfassen, die im Randbereich der Tellerscheiben **(41)** angeordnet sind und Zentrierstufen aufweisen, und dass die Tellerscheiben **(41)** durch parallel zu den Distanzhülsen **(47)** verlaufende Spannelemente zusammengehalten sind.

5. Klimaschrank nach Patentanspruch **1**,
**dadurch gekennzeichnet,**
dass die Positionierelemente **(51)** durch Schlitze in der Tellerscheibe **(41)** gebildet sind und entlang der Schlitze aus der Tellerscheibenfläche hochgebogene Laschen **(51b-51d)** umfasssen.

6. Klimaschrank nach Patentanspruch **1**,
**dadurch gekennzeichnet,**
dass die Positionierelemente **(51)** senkrecht in die Tellerscheibe **(41)** gepresste Stifte **(51e-51f)** sind.

7. Klimaschrank nach Patentanspruch **1**,
**dadurch gekennzeichnet,**
dass Mittel im Positionierantrieb **(5)** vorgesehen sind, um die Drehrichtung des Karussells **(4)** intermittierend zu steuern.

8. Klimaschrank nach Patentanspruch **7**,
**dadurch gekennzeichnet,**
dass die Mittel so ausgebildet sind, dass der Drehrichtungsänderung eine Rotationsbewegung überlagerbar ist.

9. Klimaschrank nach Patentanspruch **1**,
**dadurch gekennzeichnet,**
dass die Bedienelemente einen Positionswähler **(80)** zur Vorgabe der gewünschten Halteposition des Karussells und wenigstens eine Signallampe **(81, 82)** und/oder Signalsummer zur Signalisierung der Beendigung des Positioniervorgangs aufweisen.

10. Klimaschrank nach Patentnspruch **1**,
**dadurch gekennzeichnet,**
dass die Bedienelemente ein übergeordnetes System beinhalten, das einerseits einen Terminkalender und andereseits ein Inhaltsverzeichnis für die Lagerstücke **(50)** enthält und dass Mittel im übergeordneten System vorgesehen sind, die entsprechende Zuordnungen erlauben.

11. Klimaschrank nach Patentanspruch **1**,
**dadurch gekennzeichnet,**
dass der Positionierantrieb **(5)** innerhalb des Nutztraumes **(11)** des Klimaschrankes **(1)** angeordnet ist. **(Fig. 3a)**.

12. Klimaschrank nach Patentanspruch **1**,
**dadurch gekennzeichnet**,
dass der Positionierantrieb **(5)** ausserhalb des Nutzraumes **(11)** des Klimaschrankes **(1)** angeordnet ist. **(Fig. 3b).**

13. Karussell zur Aufnahme einer Vielzahl von Lagergutstücken auf mindestens zwei konzentrisch übereinander an einer Trägerwelle angeordneten Tellerscheiben, wobei jeweils zwei benachbarte Tellerscheiben durch mindestens drei senkrecht zwischen ihnen angeordnete sie distanzierende Teile wie Distanzhülsen oder -röhrchen beabstandet sind,
**dadurch gekennzeichnet,**
dass die Distanzhülsen mindestens drei im äusseren Bereich der Tellerscheiben **(41)** angeordnete Distanzhülsen **(47)** umfassen, von denen jede einen, durch eine Stützbohrung **(48)** einer der angrenzenden Tellerscheiben **(41)** geführten, Zentrierkragen **(47c)** sowie eine Zentrierbohrung **(47b)** zur Aufnahme des Zentrierkragens einer benachbarten Distanzhülse besitzt, wobei der Aussendurchmesser des Zentrierkragens **(47c)** dem Durchmesser der Zentrierbohrung **(47b)** und dem Durchmesser der Stützbohrung **(48)** entspricht, und wobei die Tellerscheiben **(41)** durch quer zu ihnen, in Längsbohrungen **(47a)** der Distanzhülsen **(47)** angeordnete Spannelemente **(42)** zusammengehalten sind.

14. Karussell nach Patentanspruch **13**,
**dadurch gekennzeichnet,**
die Distanzhülsen **(47)** im Peripheriebereich der Tellerscheiben **(41)** angeordnet sind.

15. Karussell nach Patentanspruch **13**,
**dadurch gekennzeichnet,**
dass die Distanzröhrchen **(46)** konzentrisch zur Trägerwelle **(45)** angeordnet sind.

16. Karussell nach Patentanspruch **13**,
**dadurch gekennzeichnet,**
dass die Tellerscheiben **(41)** Positionierelemente **(52)** zum Positionieren der Lagergutstücke **(50)** besitzen.

17. Karussell nach Patentanspruch **13**,
**dadurch gekennzeichnet,**
dass es in einer Lagervorrichtung **(13)** lagerbar und über eine lösbare Kupplung **(9, 9b)** mit einem Positionierantrieb **(5)** verbindbar ist.

18. Verwendung eines Karussells nach mindestens einem der Patentansprüche **13 - 17** in einer Einrichtung zum Behandeln von Lagergutstücken bzw. deren Inhalt,
**dadurch gekennzeichnet,**
dass die Einrichtung ein Klimaschrank **(1)** nach mindestens einem der Patentansprüche **1 - 12** ist.

## Claims

1. Conditioning cabinet, which is automatically loaded by a main system and which comprises a controllable and a conventional door as well as a revolving table which is positioned inside the conditioning cabinet and connected via a releasable coupling to a positioning drive, **characterised in that**
- the conventional door (3) is arranged at the side of the user (31), and the controllable door (2) is arranged at the side of a feeder (21);
- the controllable door (2) is, on the one hand, mounted on a first guide system (6) so as to be movable vertically to the side of the feeder (21) and, on the other hand, mounted on a second guide system (7) so as to be movable parallel to the side of a feeder (21);
- the revolving table (4) is supported on a mounting (13) arranged in the base of the interior of the conditioning cabinet;
- the revolving table (4) is composed of a support shaft (45), at least two table discs (41) and at least three spacer sleeves (47), and the support shaft (45) and the spacer sleeves (47) are arranged vertically on the table discs (41);
- on the table discs (41) are provided positioning elements (51) composed of the table surface;
- the coupling (9) between the conditioning cabinet (4) and the positioning drive (5) is positioned inside the conditioning cabinet;
- the positioning drive (5) can be controlled both by the user via operating elements and by the main system.

2. Conditioning cabinet according to Patent Claim 1, **characterised in that** the guide systems (6, 7) are linear guide systems, and the first linear guide system (6) is arranged to be movable vertically to the side of a feeder (21) on a second linear guide system (7) which is positioned parallel to the side of a feeder (21).

3. Conditioning cabinet according to Patent Claim 1, **characterised in that** the first guide system (6) is a rotary guide system and the second guide system (7) is a linear guide system, and the rotary guide system moves the controllable door (2) vertically to the side of a feeder (21) and is positioned on the second linear guide system (7) which is arranged parallel to the side of a feeder (21).

4. Conditioning cabinet according to Patent Claim 1, **characterised in that** the spacer sleeves include at least three outer spacer sleeves (47) which are arranged in the edge area of the table discs (41) and comprise centring steps, and the table discs (41) are held together by clamping elements which extend parallel to the spacer sleeves (47).

5. Conditioning cabinet according to Patent Claim 1, **characterised in that** the positioning elements (51) are formed by slots in the table disc (41) and include webs (51b-51d) bent upwards from the table disc surface along the slots.

6. Conditioning cabinet according to Patent Claim 1, **characterised in that** the positioning elements (51) are pins (51e-51f) which are vertically pressed into the table disc (41).

7. Conditioning cabinet according to Patent Claim 1, **characterised in that** means for intermittent control of the rotary direction of the revolving table are provided in the positioning drive (5).

8. Conditioning cabinet according to Patent Claim 7, **characterised in that** the means are designed in such a manner that a rotary movement is superposable over a change in rotary direction.

9. Conditioning cabinet according to Patent Claim 1, **characterised in that** the operating elements comprise a position selector (80) for entering the desired stop position of the revolving table and at least one signal lamp (81, 82) and/or signal buzzer for signalling completion of a positioning process.

10. Conditioning cabinet according to Patent Claim 1, **characterised in that** the operating elements include a main system which includes, on the one hand, a time schedule and, on the other hand, an inventory of the stored goods (50), and the main system is provided with means which permit respective associations.

11. Conditioning cabinet according to Patent Claim 1, **characterised in that** the positioning drive (5) is positioned inside the service area (11) of the conditioning cabinet (1)(Fig. 3a).

12. Conditioning cabinet according to Patent Claim 1, **characterised in that** the positioning drive (5) is positioned outside the service area (11) of the conditioning cabinet (1)(Fig. 3b).

13. Revolving table for accommodating a plurality of stored items on at least two table discs which are concentrically stacked on a carrier shaft, and two respective adjacent table discs are spaced by at least three elements, such as spacing sleeves or spacing tubes, which are arranged vertically thereinbetween and distancing them, **characterised in that** the spacing sleeves include at least three spacing sleeves (47) arranged in the outer area of the table discs (41), each having a centring collar (47c) which is guided through a support bore (48) of one of the adjacent table discs (41), as well as a centring bore (47b) for accommodating the centring collar of an adjacent spacing sleeve, and the outside diameter of the centring collar (47c) corresponds with the diameter of the centring bore (47b) and the diameter of the support bore (48), and the table discs (41) are held together by clamping elements (42) arranged transversely to the latter and arranged in longitudinal bores (47a) of the spacing sleeves (47).

14. Revolving table according to Patent Claim 13, **characterised in that** the spacing sleeves (47) are positioned in the peripheral area of the table discs (41).

15. Revolving table according to Patent Claim 13, **characterised in that** the spacing tubes (46) are positioned concentrically to the support shaft (45).

16. Revolving table according to Patent Claim 13, **characterised in that** the table discs (41) have positioning elements (52) for positioning the stored items (50).

17. Revolving table according to Patent Claim 13, **characterised in that** it can be mounted in a storage device (13) and connected via a releasable coupling (9, 9b) to a positioning drive (5).

18. Use of a revolving table according to at least one of Patent Claims 13 - 17 in a device for treating stored goods or their contents, **characterised in that** the device is a conditioning cabinet (1) according to at least one of Patent Claims 1 - 12.

## Revendications

1. Armoire climatique pouvant être chargée automatiquement par un système supérieur et comprenant une porte commandée et une porte conventionnelle, ainsi qu'un carrousel situé à l'intérieur de l'armoire climatique, relié à un entraînement de positionnement par l'intermédiaire d'un accouplement démontable, caractérisée en ce que
- la porte conventionnelle (3) est disposée d'un côté utilisateur (31) et la porte commandée (2) est disposée d'un côté chargement (21),
- la porte commandée (2) est logée de façon mobile, d'une part dans un premier système de guidage (6), perpendiculairement au côté chargement (21), et d'autre part dans un second système de guidage (7), parallèlement au côté chargement (21),
- le carrousel (4) est supporté par un palier (13) aménagé dans le fond de l'intérieur de l'armoire climatique,
- le carrousel (4) est constitué d'un arbre de support (45), d'au moins deux disques-plateaux (41) et d'au moins trois douilles entretoises (47), et l'arbre de support (45) et les douilles entretoises (47) sont disposés perpendiculairement aux et sur les disques-plateaux (41),
- des éléments de positionnement (51) montés sur la surface du disque-plateau (41) sont prévus,
- l'accouplement (9) entre le carrousel (4) et l'entraînement de positionnement (5), est disposé à l'intérieur de l'armoire climatique, et que
- l'entraînement de positionnement (5) peut être commandé à la fois par l'utilisateur, par l'intermédiaire d'éléments de commande, et par le système supérieur.

2. Armoire climatique selon la revendication 1, caractérisée en ce que les systèmes de guidage (6, 7) sont des systèmes de guidage linéaire, le premier système de guidage linéaire (6) étant aménagé perpendiculairement au côté chargement (21), dans le second système de guidage linéaire (7) aménagé parallèlement au côté chargement (21).

3. Armoire climatique selon la revendication 1, caractérisée en ce que le premier système de guidage (6) est un système de guidage en rotation, et le second système de guidage (7) est un système de guidage linéaire, le système de guidage en rotation déplaçant la porte commandée (2) perpendiculairement au côté chargement (21) et étant installé dans le second système de guidage linéaire (7) disposé parallèlement au côté chargement (21).

4. Armoire climatique selon la revendication 1, caractérisée en ce que les douilles entretoises comprennent au moins trois douilles entretoises extérieures (47) disposées dans une zone marginale des disques-plateaux (41) et comportant des rebords de centrage, et en ce que les disques-plateaux (41) sont maintenus ensemble au moyen d'éléments de serrage s'étendant parallèlement aux douilles entretoises (47).

5. Armoire climatique selon la revendication 1, caractérisée en ce que les éléments de positionnement (51) sont constitués de fentes ménagées dans le disque-plateau (41) et comportent des languettes (51b à 51d) repliées vers le haut le long des fentes, à partir de la surface du disque-plateau.

6. Armoire climatique selon la revendication 1, caractérisée en ce que les éléments de positionnement (51) sont des goupilles (51e à 51f) emmanchées perpendiculairement, avec serrage, dans le disque-plateau (41).

7. Armoire climatique selon la revendication 1, caractérisée en ce que des moyens sont prévus dans l'entraînement de positionnement, pour commander de façon intermittente le sens de rotation du carrousel (4).

8. Armoire climatique selon la revendication 7, caractérisée en ce que les moyens sont configurés de manière telle qu'un mouvement de rotation puisse être superposé aux changements de sens de rotation.

9. Armoire climatique selon la revendication 1, caractérisée en ce que les éléments de commande comportent un sélecteur de position (80) permettant de prédéfinir la position d'arrêt souhaitée du carrousel, et au moins un témoin lumineux (81, 82) et/ ou un ronfleur témoin destinés à signaler la fin de l'opération de positionnement.

10. Armoire climatique selon la revendication 1, caractérisée en ce que les éléments de commande comprennent un système supérieur qui contient d'une part un programme chronologique et d'autre part un inventaire des objets à stocker (50), et en ce que des moyens existent dans le système supérieur, qui permettent d'établir des associations correspondantes.

11. Armoire climatique selon la revendication 1, caractérisée en ce que l'entraînement de positionnement (5) est disposé à l'intérieur de l'espace utile (11) de l'armoire climatique (1). (Figure 3a).

12. Armoire climatique selon la revendication 1, caractérisée en ce que l'entraînement de positionnement (5) est disposé à l'extérieur de l'espace utile (11) de l'armoire climatique (1). (Figure 3b).

13. Carrousel destiné à recevoir une multitude d'éléments d'objets à stocker sur au moins deux disques-plateaux disposés concentriquement l'un au-dessus de l'autre sur un arbre de support, deux disques-plateaux contigus étant respectivement éloignés l'un de l'autre au moyen d'au moins trois éléments tels que des douilles entretoises ou des petits tubes entretoises disposés verticalement entre eux, les maintenant à une distance l'un de l'autre,
caractérisé en ce que les douilles entretoises comprennent au moins trois douilles entretoises (47) disposées dans la partie extérieure des disques-plateaux (41), ces douilles comprenant chacune une collerette de centrage (47c) guidée dans un alésage de soutien de l'un des disques-plateaux contigus (41), ainsi qu'un alésage de centrage (47b) destiné à recevoir la collerette de centrage d'une douille entretoise contiguë, le diamètre extérieur de la collerette de centrage (47c) correspondant au diamètre de l'alésage de centrage (47b) et au diamètre de l'alésage de soutien (48), et les disques-plateaux (41) étant maintenus ensemble au moyen d'éléments de serrage (42) disposés transversalement aux disques dans des alésages longitudinaux (47a) des douilles entretoises (47).

14. Carrousel selon la revendication 13, caractérisé en ce que les douilles entretoises (47) sont disposées dans la zone périphérique des disques-plateaux (41).

15. Carrousel selon la revendication 13, caractérisé en ce que les petits tubes entretoises (46) sont disposés concentriquement à l'arbre de support (45).

16. Carrousel selon la revendication 13, caractérisé en ce que les disques-plateaux (41) possèdent des éléments de positionnement (52) permettant de positionner les éléments d'objets à stocker (50).

17. Carrousel selon la revendication 13, caractérisé en ce qu'il peut être logé dans un dispositif de palier (13) et peut être relié à un entraînement de positionnement (5) via un accouplement démontable (9, 9b).

18. Utilisation d'un carrousel, selon l'une au moins des revendications 13 à 17, dans un dispositif de traitement d'éléments d'objets à stocker ou de leur contenu, caractérisée en ce que ledit dispositif est une armoire climatique (1) selon l'une au moins des revendications 1 à 12.
